(19) Europäisches Patentamt
European Patent Office
Office européen des brevets

(11) Veröffentlichungsnummer: **0 281 731**
**A2**

# (12) EUROPÄISCHE PATENTANMELDUNG

(21) Anmeldenummer: **88100649.8**

(22) Anmeldetag: **19.01.88**

(51) Int. Cl.[4]: **C07D 207/34**, C07D 401/12, C07D 405/12, C07D 409/12, A01N 43/36

(30) Priorität: **31.01.87 DE 3702852**

(43) Veröffentlichungstag der Anmeldung:
**14.09.88 Patentblatt 88/37**

(84) Benannte Vertragsstaaten:
**AT BE CH DE ES FR GB GR IT LI NL**

(71) Anmelder: **BAYER AG**
**D-5090 Leverkusen 1, Bayerwerk(DE)**

(72) Erfinder: **Wollweber, Detlef, Dr.**
**Kirschbaumstrasse 34**
**D-5600 Wuppertal 1(DE)**
Erfinder: **Krämer, Wolfgang, Dr.**
**Rosenkranz 25**
**D-5093 Burscheid 2(DE)**
Erfinder: **Brandes, Wilhelm, Dr.**
**Eichendorffstrasse 3**
**D-5653 Leichlingen(DE)**
Erfinder: **Dutzmann, Stefan, Dr.**
**Leinenweberweg 33**
**D-4000 Düsseldorf 13(DE)**
Erfinder: **Hänssler, Gerd, Dr.**
**Am Arenzberg 58a**
**D-5090 Leverkusen 3(DE)**

## (54) 1-Aminomethyl-3-aryl-4-cyano-pyrrole.

(57) 1-Aminomethyl-3-aryl-4-cyano-pyrrole der allgemeinen Formel (I),

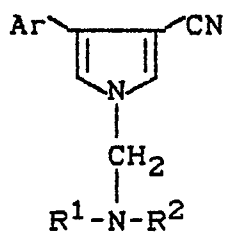

(I)

in welcher
Ar für gegebenenfalls substituiertes Phenyl steht,
$R^1$ für gegebenenfalls substituiertes Alkyl, für Alkenyl, Alkinyl oder Cycloalkyl oder für jeweils gegebenenfalls substituiertes Aralkyl oder Aryl steht und
$R^2$ für jeweils gegebenenfalls substituiertes Heterocyclylalkyl, Heterocyclylalkenyl, Heterocyclylalkinyl oder Heterocyclyl, für Dialkylaminoalkyl oder für jeweils gegebenenfalls substituiertes Cycloalkylalkyl oder Phenethyl steht und ihre Verwendung zur Bekämpfung von Schädlingen.
Die neuen 1-Aminomethyl-3-aryl-4-cyano-pyrrole der allgemeinen Formel (I) können nach Analogieverfahren

EP 0 281 731 A2

hergestellt werden, z.B. indem man geeignete 3-Aryl-4-cyano-pyrrole mit Formaldehyd und geeigneten Aminen umsetzt.

# 1-Aminomethyl-3-aryl-4-cyano-pyrrole

Die Erfindung betrifft neue 1-Aminomethyl-3-aryl-4-cyano-pyrrole, mehrere Verfahren zu ihrer Herstellung sowie ihre Verwendung als Schädlingsbekämpfungsmittel.

Es ist bereits bekannt, daß bestimmte Dithiocarbamate, wie beispielsweise das Zink-ethylen-1,2-bis-(dithiocarbamat) oder das Mangan-ethylen-1,2-bis-(dithiocarbamat) gute fungizide Eigenschaften besitzen (vgl. z.B. K. H. Büchel, "Pflanzenschutz und Schädlingsbekämpfung", S, 137, 138, Thieme Verlag, Stuttgart 1977). Weiterhin ist bekannt, daß bestimmte Sulfenamide, wie beispielsweise das N,N-Dimethyl-N'-phenyl-N-(fluordichlormethylsulfenyl)-sulfamid insbesondere gegen Botrytis-Arten hervorragend wirksam sind (vgl. z. B. K. H. Büchel "Pflanzenschutz und Schädlingsbekämpfung", S. 141, Thieme Verlag Stuttgart 1977).

Die Wirksamkeit dieser vorbekannten Verbindungen ist jedoch insbesondere bei niedrigen Aufwandmengen und Konzentrationen nicht in allen Anwendungsbereichen völlig zufriedenstellend.

Weiterhin ist bekannt, daß bestimmte 3-Aryl-pyrrole, wie beispielsweise das 4-Cyano-3-(2,3-dichlorphenyl)-pyrrol ebenfalls gute fungizide Eigenschaften besitzen (vgl. z.B. EP 174 910 oder EP 182 738 oder EP 133 247).

Es wurden neue 1-Aminomethyl-3-aryl-4-cyano-pyrrole der allgemeinen Formel (I),

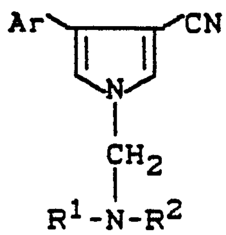

(I)

in welcher
Ar für gegebenenfalls substituiertes Phenyl steht,
$R^1$ für gegebenenfalls substituiertes Alkyl, für Alkenyl, Alkinyl oder Cycloalkyl oder für jeweils gegebenenfalls substituiertes Aralkyl oder Aryl steht und
$R^2$ für jeweils gegebenenfalls substituiertes Heterocyclylalkyl, Heterocyclylalkenyl, Heterocyclylalkinyl oder Heterocyclyl, für Dialkylaminoalkyl oder für jeweils gegebenenfalls substituiertes Cycloalkylalkyl oder Phenethyl steht,
gefunden.

Weiterhin wurde gefunden, daß man die neuen 1-Aminomethyl-3-aryl-4-cyano-pyrrole der allgemeinen Formel (I),

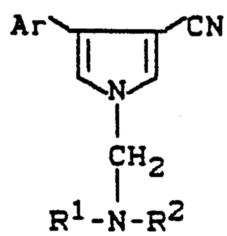

(I)

in welcher
Ar für gegebenenfalls substituiertes Phenyl steht,
$R^1$ für gegebenenfalls substituiertes Alkyl, für Alkenyl, Alkinyl oder Cycloalkyl oder für jeweils gegebenenfalls substituiertes Aralkyl oder Aryl steht und
$R^2$ für jeweils gegebenenfalls substituiertes Heterocyclylalkyl, Heterocyclylalkenyl, Heterocyclylalkinyl oder Heterocyclyl, für Dialkylaminoalkyl oder für jeweils gegebenenfalls substituiertes Cycloalkylalkyl oder Phenethyl steht,
erhält, wenn man
(a) 3-Aryl-4-cyano-pyrrole der Formel (II),

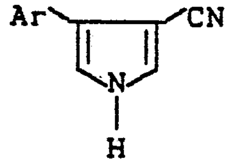

(II)

in welcher
Ar die oben angegebene Bedeutung hat,
mit Formaldehyd und Aminen der Formel (III),

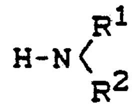

(III)

in welcher
$R^1$ und $R^2$ die oben angegebene Bedeutung haben,
gegebenenfalls in Gegenwart eines Verdünnungsmittels und gegebenenfalls in Gegenwart eines Reaktionshilfsmittels umsetzt, oder wenn man
(b) 3-Aryl-4-cyano-pyrrole der Formel (IV),

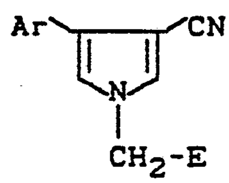

(IV)

in welcher
Ar die oben angegebene Bedeutung hat und
E für eine elektronenanziehende Abgangsgruppe steht,
mit Aminen der Formel (III),

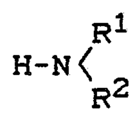

(III)

in welcher
$R^1$ und $R^2$ die oben angegebene Bedeutung haben,
gegebenenfalls in Gegenwart eines Verdünnungsmittels und gegebenenfalls in Gegenwart eines Säurebindemittels umsetzt.

Schließlich wurde gefunden, daß die neuen 1-Aminomethyl-3-aryl-4-cyano-pyrrole der allgemeinen Formel (I) eine gute Wirkung gegen Schädlinge besitzen.

Überraschenderweise zeigen die erfindungsgemäßen 1-Aminomethyl-3-aryl-4-cyano-pyrrole der allgemeinen Formel (I) neben einer besseren fungiziden Wirksamkeit im Vergleich zu den aus dem Stand der Technik vorbekannten Dithiocarbamaten oder Sulfenamiden, wie beispielsweise das Zink-ethylen-1,2-bis-(dithiocarbamat) oder das Mangan-ethylen-1,2-bis-(dithiocarbamat) oder das N,N-Dimethyl-N'-phenyl-N'-phenyl-N'-(fluordichlormethylsulfenyl)-sulfamid gleichzeitig eine deutlich verbesserte Kulturpflanzenverträglichkeit im Vergleich zu den aus dem Stand der Technik vorbekannten 3-Aryl-pyrrolen, wie beispielsweise das 4-Cyano-3-(2,3-dichlor-phenyl)-pyrrol, welches chemisch und/oder wirkungsmäßig naheliegende Verbindungen sind.

Die erfindungsgemäßen 1-Aminomethyl-3-aryl-4-cyano-pyrrole sind durch die Formel (I) allgemein definiert. Bevorzugt sind Verbindungen der Formel (I), bei welchen
Ar für gegebenenfalls einfach bis mehrfach, gleich oder verschieden substituiertes Phenyl steht, wobei als Substituenten infrage kommen: Halogen, jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,
$R^1$ für gegenbenefalls substituiertes, geradkettiges oder verzweigtes Alkyl mit 1 bis 6 Kohlenstoffatomen steht, wobei als Substituenten infrage kommen: Cyano, Cycloalkyl mit 3 bis 7 Kohlenstoffatomen, jeweils geradkettiges oder verzweigtes Alkoxy, Alkylcarbonyl, Alkoxycarbonyl, Alkylaminocarbonyl, Dialkylaminocarbonyl, Alkylsulfinyl, Alkylsulfonyl oder Dialkylamino mit jeweils 1 bis 6 Kohlenstoffatomen in den einzelnen Alkylteilen; außerdem für jeweils geradkettiges oder verzweigtes Alkenyl oder Alkinyl mit jeweils 3 bis 6 Kohlenstoffatomen, für Cycloalkyl mit 3 bis 7 Kohlenstoffatomen oder für jeweils gegebenenfalls einfach bis mehrfach, gleich oder verschieden im Phenylteil substituiertes Phenyl oder Benzyl steht, wobei als Substituenten jeweils infrage kommen: Halogen, Cyano, jeweils geradkettiges oder verzweigtes Alkyl, Alkoxy, Halogenalkyl, Halogenalkoxy, Alkylcarbonyl oder Alkoxycarbonyl mit jeweils 1 bis 6 Kohlenstoffatomen in den einzelnen Alkylteilen und im Fall des Halogenalkyl-oder Halogenalkoxy-Restes mit jeweils 1 bis 9 gleichen oder verschiedenen Halogenatomen und
$R^2$ für jeweils geradkettiges oder verzweigtes Heterocyclylalkyl mit 1 bis 6 Kohlenstoffatomen im Alkylteil, Heterocyclylalkenyl mit 3 bis 6 Kohlenstoffatomen im Alkenylteil, Heterocyclylakinyl mit 3 bis 6 Kohlenstoffatomen im Alkinylteil oder Heterocyclyl steht, wobei Heterocyclyl jeweils für einen 5-bis 7-gliedrigen, gegebenenfalls einfach bis mehrfach, gleich oder verschieden substituierten, ungesättigen oder gesättigten Heterocyclus mit 1 bis 3 Heteroatomen, insbesondere Stickstoff, Sauerstoff oder Schwefel steht und wobei als Subkstituenten jeweils infrage kommen: Halogen, Cyano,
jeweils geradkettiges oder verzweigtes Alkyl, Alkoxy, Halogenalkyl, Halogenalkoxy, Alkylcarbonyl oder Alkoxycarbonyl mit jeweils 1 bis 6 Kohlenstoffatomen in den einzelnen Alkylteilen und im Fall des Halogenalkyl-oder Halogenalkoxy-Restes mit jeweils 1 bis 9 gleichen oder verschiedenen Halogenatomen; außerdem für geradkettiges oder verzweigtes Dialkylaminoalkyl mit jeweils 1 bis 6 Kohlenstoffatomen in den einzelnen Alkylteilen steht, ferner für im Cycloalkylteil gegebenenfalls einfach bis mehrfach, gleich oder verschieden durch Halogen und/oder geradkettiges oder verzweigtes Alkyl mit 1 bis 4 Kohlenstoffatomen substituiertes Cycloalkylalkyl mit 1 bis 6 Kohlenstoffatomen im geradkettigen oder verzweigten Alkylteil und 3 bis 7 Kohlenstoffatomen im Cycloalkylteil oder für im Phenylteil gegebenenfalls einfach bis mehrfach, gleich oder verschieden durch Cyano, Halogen und/oder jeweils geradkettiges oder verzweigtes Alkyl oder Alkoxy mit jeweils 1 bis 4 Kohlenstoffatomen substituiertes Phenylethyl steht.

Besonders bevorzugt sind Verbindungen der Formel (I), bei welchen
Ar für gegebenenfalls ein-bis dreifach, gleich oder verschieden substituiertes Phenyl steht, wobei als Substituenten infrage kommen: Fluor, Chlor, Brom, Methyl, Ethyl, n-oder i-Propyl, n-, i-, s-oder t-Butyl, Methoxy, Ethoxy, Methylthio, Trifluormethyl, Trifluormethoxy oder Trifluormethylthio,
$R^1$ für jeweils gegebenenfalls substituiertes Methyl, Ethyl oder n-oder i-Propyl sowie n-, i-oder s-Butyl steht, wobei als Substituenten jeweils infrage kommen: Cyano, Methoxy, Ethoxy, Methylcarbonyl, Ethylcarbonyl, Methoxycarbonyl, Ethoxycarbonyl, Methylaminocarbonyl, Dimethylaminocarbonyl, Ethylaminocarbonyl, Diethylaminocarbonyl, Methylsulfinyl, Methylsulfonyl, Dimethylamino, Diethylamino oder Dipropylamino, Dibutylamino oder Cyclohexyl; außerdem für Allyl, n-oder i-Butenyl, Propargyl, n-oder i-Butinyl, für Cyclopentyl, Cyclohexyl, Cyclopropyl oder für jeweils gegebenenfalls ein-bis dreifach, gleich oder verschieden im Phenylteil substituiertes Benzyl oder Phenyl steht, wobei als Substituenten infrage kommen: Fluor, Chlor, Brom, Cyano, Methyl, Ethyl, n-oder i-Propyl, n-, i-oder s-Butyl, Methoxy, Ethoxy, Trifluormethyl, Trifluormethoxy, Acetyl, Methoxycarbonyl oder Ethoxycarbonyl und
$R^2$ für jeweils gegebenenfalls im Heterocyclylteil ein-oder zweifach, gleich oder verschieden substituiertes Heterocyclylmethyl, Heterocyclylethyl oder Heterocyclylpropyl steht, wobei als Heterocyclylrest jeweils einer der folgenden Reste infrage kommt

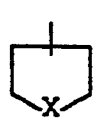 ; 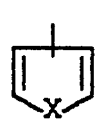 ; 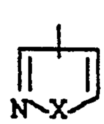 ; 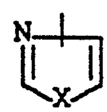 ; 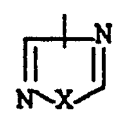 ; 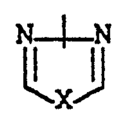

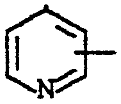 ; 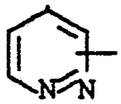 ; 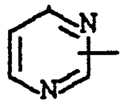 ; 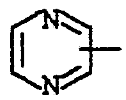 ; 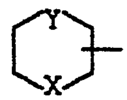

und 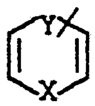 ,

wobei X jeweils für Sauerstoff, Schwefel oder eine NH-Gruppe steht und Y jeweils für Sauerstoff, Schwefel, eine NH-Gruppe oder eine $CH_2$-Gruppe steht und wobei als Substituenten jeweils infrage kommen: Fluor, Chlor, Brom, Cyano, Methyl, Ethyl, n-oder i-Propyl, n-, i-, s-oder t-Butyl, Methoxy, Ethoxy, Trifluormethyl, Trifluormethoxy, Acetyl, Methoxycarbonyl oder Ethoxycarbonyl; außerdem für Dimethylaminoethyl, Diethylaminoethyl, Diemthylaminopropyl, Diethylaminopropyl, Dipropylaminopropyl, Dibutylaminopropyl, Dibutylaminobutyl, Dibutylaminoethyl, Dipropylaminoethyl, Dimethylaminobutyl, Diethylaminobutyl, Dimethylaminopentyl, Diethylaminopentyl, für jeweils gegebenenfalls ein-bis dreifach, gleich oder verschieden durch Methyl, Ethyl und/oder Isopropyl substituiertes Cyclohexylmethyl oder Cyclohexylethyl oder für gegebenenfalls ein-bis dreifach, gleich oder verschieden durch Fluor, Chlor, Brom, Cyano, Methyl, Ethyl, n-oder i-Propyl, n-, i-, s-oder t-Butyl, Methoxy, Ethoxy, n-oder i-Propoxy und n-, i-, s-oder t-Butoxy substituiertes Phenethyl steht.

Ganz besonders bevorzugt sind Verbindungen der Formel (I), bei welchen
Ar für zweifach durch Chlor substituiertes Phenyl steht,
$R^1$ für Methyl, Ethyl, n-oder i-Propyl, n-, i-oder s-Butyl, Allyl, Propargyl, für Cyclohexyl, Cyclopropyl, Cyclopentyl, Cyclohexylmethyl, Phenyl, Benzyl oder für Cyanethyl steht und
$R^2$ für jeweils gegebenenfalls ein-bis dreifach, gleich oder verschieden durch Chlor und Methyl substituiertes Heterocyclylmethyl, Heterocyclylethyl oder Heterocyclylpropyl siteht, wobei als Heterocyclylreste infrage kommen:

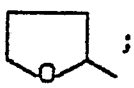 ; 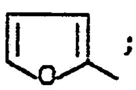 ; 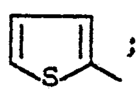 ; 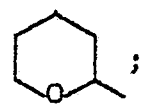 ; 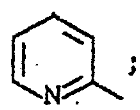 ;

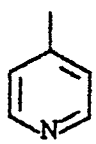 oder 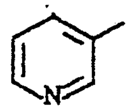 ;

oder für jeweils gegebenenfalls ein-bis dreifach, gleich oder verschieden durch Methyl und Ethyl substituiertes 2-Heterocyclylethyl, 2-Heterocyclylpropyl oder 3-Heterocyclylpropyl steht, wobei als Heterocyclylreste infrage kommen:

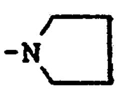 ; 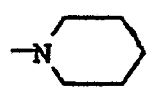 ; 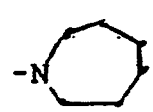 ; 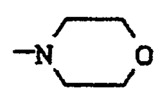 ;

außerdem für Diethylaminoethyl, Dimethylaminopropyl, Diethylaminopropyl, Dibutylaminopropyl oder Diethylaminopentyl, für Cyclcohexylmethyl oder für Phenethyl steht.

Insbesonders bevorzugt sind Verbindungen der Formel (I), bei welchen
Ar für 2,3-Dichlorphenyl steht,
$R^1$ für Cyanethyl steht und
$R^2$ für jeweils gegebenenfalls ein-bis dreifach, gleich oder verschieden durch Chlor und Methyl substituiertes Heterocyclylmethyl, Heterocyclylethyl oder Heterocyclylpropyl steht, wobei als Heterocyclylreste infrage kommen:

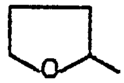 ; 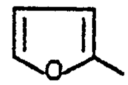 ; 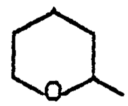 ; 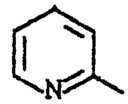 ; 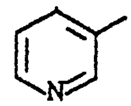

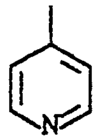 ;

oder für jeweils gegebenenfalls ein-bis dreifach, gleich oder verschieden durch Methyl und Ethyl substituiertes 2-Heterocyclylethyl, 2-Heterocyclylpropyl oder 3-Heterocyclylpropyl steht, wobei als Heterocyclylreste infrage kommen:

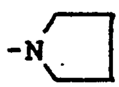 ; 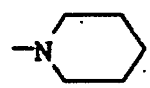 ; 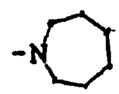 oder 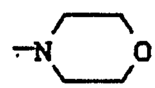 ;

außerdem für Diethylaminoethyl, Dimethylaminopropyl, Diethylaminopropyl, Dibutylaminopropyl oder Diethylaminopentyl, für Cyclohexylmethyl oder Phenethyl steht.

Insbesonders bevorzugt sind daneben auch Verbindungen der Formel (I), bei welchen
Ar für 2,3-Dichlorphenyl steht,
$R^1$ für Methyl, Ethyl, n-oder i-Propyl, n-, i-oder s-Butyl, Allyl, Propargyl, für Cyclcopropyl, Cyclopentyl, Cyclohexyl, Cyclohexylmethyl, Phenyl, Benzyl oder Cyanoethyl steht und
$R^2$ für gegebenenfalls ein-bis dreifach, gleich oder verschieden durch Chlor und Methyl substituiertes Heterocyclylmethyl steht, wobei als Heterocyclylreste infrage kommen:

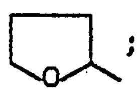 ; 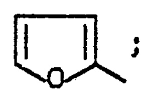 ; 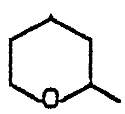 ; 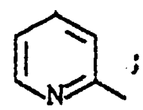 ; 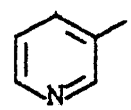 oder

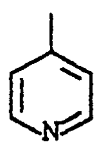 .

Im einzelnen seien außer den bei den Herstellungsbeispielen genannten Verbindungen die folgenden 1-Aminomethyl-3-aryl-4-cyano-pyrrole der allgemeinen Formel (I) genannt:

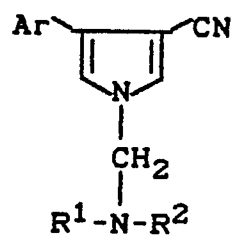 (I)

| Ar | 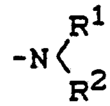 |
|---|---|
| 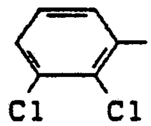 | 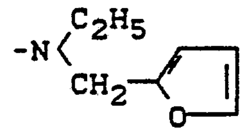 |
| 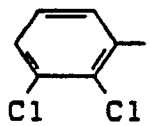 | 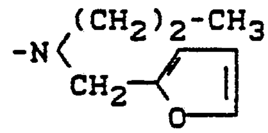 |
| 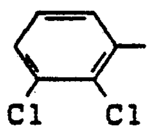 | 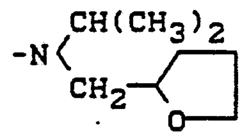 |
| 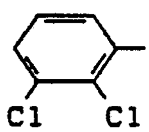 | 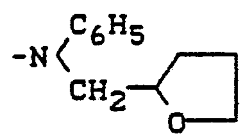 |
| 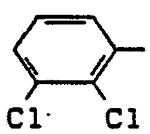 | 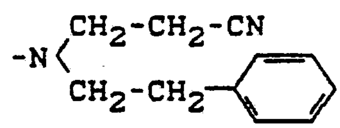 |
| 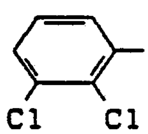 | 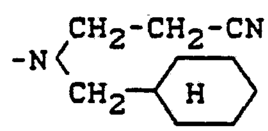 |
| 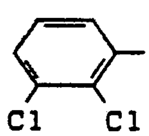 | 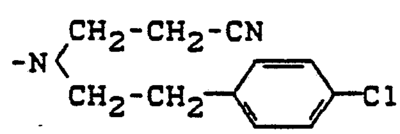 |

| Ar | 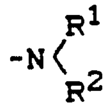 |
| --- | --- |
| 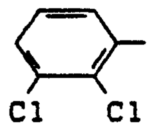 | 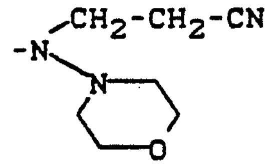 |
| 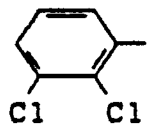 | 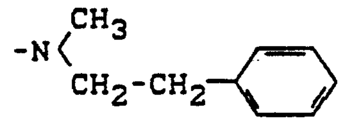 |
| 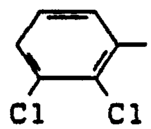 | 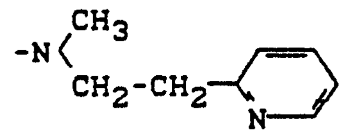 |
| 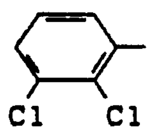 | 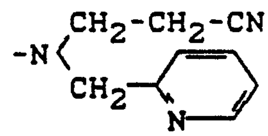 |
| 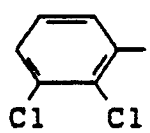 | 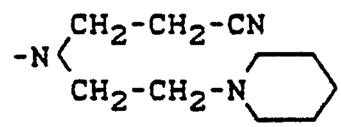 |
| 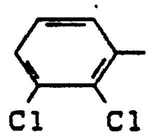 | 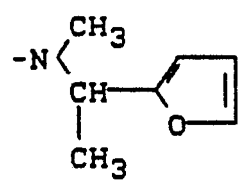 |
| 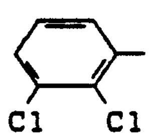 | 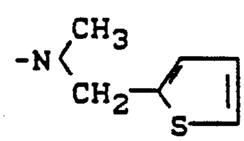 |

| Ar | 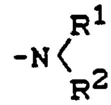 |
|---|---|
| 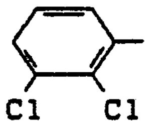 | 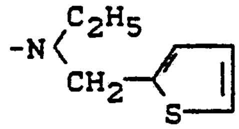 |
| 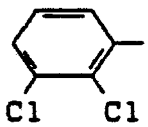 | 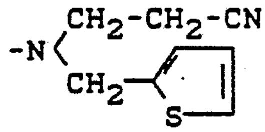 |
| 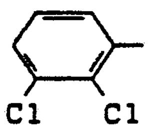 | 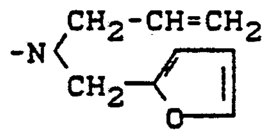 |
| 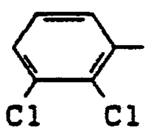 | 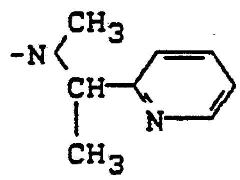 |
| 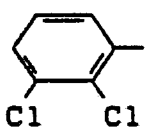 | 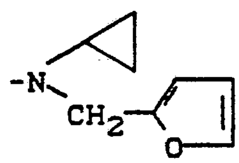 |
| 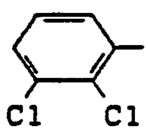 | 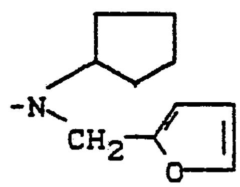 |

Verwendet man beispielsweise 4-Cyano-3-(2,3-dichlorphenyl)-pyrrol, Formaldehyd und N-Methyl-N-(2-tetrahydrofurylmethyl)-amin als Ausgangsstoffe, so läßt sich der Reaktionsablauf des erfindungsgemäßen Verfahrens (a) durch das folgende Formelschema darstellen:

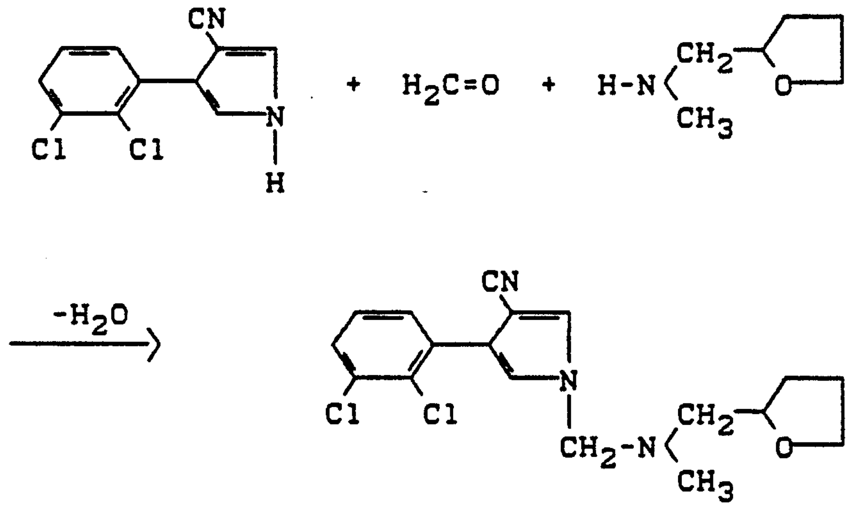


Verwendet man beispielsweise 1-Chlormethyl-4-cyano-3-(2,3-dichlorphenyl)-pyrrol und N-Ethyl-N-(2-furylmethyl)-amin als Ausgangsstoffe, so läßt sich der Reaktionsablauf des erfindungsgemäßen Verfahrens (b) durch das folgende Formelschema darstellen:

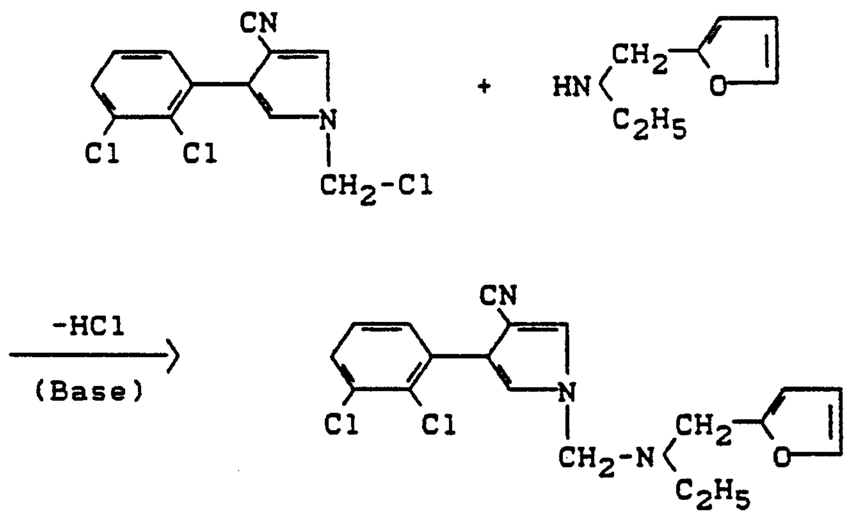


Die zur Durchführung des erfindungsgemäßen Verfahrens (a) als Ausgangsstoffe benötigten 3-Aryl-4-cyano-pyrrole sind durch die Formel (II) allgemein definiert. In dieser Formel (II) steht Ar vorzugsweise für diejenigen Reste, die bereits im Zusammenhang mit der Beschreibung der erfindungsgemäßen Stoffe der Formel (I) für diesen Substituenten genannt wurden.

Die 3-Aryl-4-cyano-pyrrole der Formel (II) sind bekannt (vgl. z.B. EP 174 910, EP 182 738 oder EP 133 247).

Die zur Durchführung des erfindungsgemäßen Verfahrens (b) als Ausgangsstoffe benötigten 3-Aryl-4-cyano-pyrrole sind durch die Formel (IV) allgemein definiert. In dieser Formel (IV) steht Ar vorzugsweise für diejenigen Reste, die bereits im Zusammenhang mit der Beschreibung der erfindungsgemäßen Stoffe der Formel (I) für diesen Substituenten genannt wurden.

E steht vorzugsweise für Hydroxy oder Halogen, insbesondere für Chlor.

Die 3-Aryl-4-cyano-pyrrole der Formel (IV) sind ebenfalls bekannt (vgl. z.B. EP 133 247).

Die zur Durchführung der erfindungsgemäßen Verfahren (a) und (b) weiterhin als Ausgangsstoffe benötigten Amine sind durch die Formel (III) allgemein definiert. In dieser Formel (III) stehen $R^1$ und $R^2$ vorzugsweise für diejenigen Reste, die bereits im Zusammenhang mit der Beschreibung der erfindungsgemäßen Stoffe der Formel (I) für diese Substituenten genannt wurden.

Die Amine der Formel (III) sind allgemein bekannte Verbindungen der organischen Chemie oder erhältlich in Analogie zu bekannten Verfahren (vgl. z.B. GB 10 31 916 vom 2.6.1966; Org. Magnet. Res. 7, 488 - 495 [1975]; Kogyo Kaguka Zasshi 63, 1593 - 1597 [1960] bzw. CA 60: 10 542 f).

Als Verdünnungsmittel zur Durchführung des erfindungsgemäßen Verfahrens (a) kommen inerte organische Lösungsmittel oder wässrige Systeme infrage. Vorzugsweise verwendet man protische Lösungsmittel, beispielsweise Alkohole, wie Methanol, Ethanol oder Propanol oder Carbonsäuren, wie Ameisensäure, Essigsäure oder Propionsäure oder deren Gemische mit Wasser. Es ist auch möglich, das erfindungsgemäße Verfahren (a) in aprotischen Lösungsmitteln duchzuführen. Hierzu gehören insbesondere alidphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldi-methyl-oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (a) wird gegebenenfalls in Gegenwart eines geeigneten Reaktionshilfmittels durchgeführt. Hierzu eignen sich entweder katalytische bis äquimolare Mengen einer organischen oder anorganischen Säure oder entsprechende Mengen einer geeigneten Base. Als saure Reaktionshilfsmittel kommen insbesondere anorganische Mineralsäuren, wie Phosphorsäure, Schwefelsäure, Salpetersäure, Chlorwasserstoffsäure oder Bromwasserstoffsäure oder organische Säuren, wie Ameisensäure, Essigsäure, Propionsäure, Methansulfonsäure, Benzolsulfonsäure oder Toluolsulfonsäure infrage.

Als basische Reaktionshilfsmittel kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclonen(DBN) oder Diazabicycloundecen(DBU).

Es ist jedoch auch möglich, das als Reaktionspartner verwendete Amin der Formel (III) in entsprechendem Überschuß gleichzeitig als Reaktionshilfsmittel zu verwenden.

'Die Reaktionstemperaturen können bei der Durchführung des erfindungsgemäßen Verfahrens (a) in einem größeren Bereich variiert werden. Im allgemeinen arbeitet man bei Temperaturen zwischen 0 °C und 120 °C, vorzugsweise bei Temperaturen zwischen 20 °C und 90 °C.

Zur Durchführung des erfindungsgemäßen Verfahrens (a) setzt man pro Mol an 3-Aryl-4-cyano-pyrrol der Formel (ii) im allgemeinen 1,0 bis 2,0 Mol, vorzugsweise 1,0 bis 1,5 Mol an Amin der Formel (III) und 1,0 bis 2,0 Mol, vorzugsweise 1,0 bis 1,5 Mol an Formaldehyd ein. Der Formaldehyd wird entweder in Form einer wässrigen Lösung, als Paraformaldehyd oder als 1,3,5-Trioxan eingesetzt. Vorzugsweise verwendet man eine wässrige Lösung.
Die Reaktionsdurchführung, Aufarbeitung und Isolierung der Reaktionsprodukte der Formel (I) erfolgt in Analogie zu bekannten Verfahren (vgl. EP 133 247).'

Als Verdünnungsmittel zur Durchführung des erfindungsgemäßen Verfahrens (b) kommen inerte organische Lösungsmittel infrage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl-oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (b) wird gegebenenfalls in Gegenwart eines geeigneten Säurebindemittels durchgeführt. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen(DBN) oder Diazabicycloundecen(DBU).

Es ist jedoch auch möglich, das als Reaktionspartner in Frage kommende Amin der Formel (III) in entsprechendem Überschuß gleichzeitig als Reaktionshilfsmittel einzusetzen.

Die Reaktionstemperaturen können bei der Durchführung des erfindungsgemäßen Verfahrens (b) in einem größeren Bereich variiert werden. Im allgemeinen arbeitet man bei Temperaturen zwischen 0 °C und 100 °C, vorzugsweise bei Temperaturen zwischen 20 °C und 60 °C.

Zur Durchführung des erfindungsgemäßen Verfahrens (b) setzt man pro Mol an 3-Aryl-4-cyano-pyrrol der Formel (IV) im allgemeinen 1,0 bis 2,0 Mol, vorzugsweise 1,0 bis 1,5 Mol an Amin der Formel (III) und gegebenenfalls 1,0 bis 2,0 Mol, vorzugsweise 1,0 bis 1,5 Mol an Säurebindemittel ein. Die Reaktionsdurchführung, Aufarbeitung und Isolierung der Reaktionsprodukte der Formel (I) erfolgt in Analogie zu bekannten Verfahren.

Die erfindungsgemäßen Wirkstoffe weisen eine starke Wirkung gegen Schädlinge auf und können zur

Bekämpfung von unerwünschten Schadorganismen praktisch eingesetzt werden. Die Wirkstoffe sind z.B. für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum; Phytophthora-Arten, wie beispielsweise Phytophthora infestans;

Pseudoperonospora-Arten, wie beispielsweise Pseudoperonospora humuli oder Pseudoperonospora cubensis;

Plasmopara-Arten, wie beispielsweise Plasmopara viticola;

Peronospora-Arten, wie beispielsweise Peronospora pisi oder P. brassicae;

Erysiphe-Arten, wie beispielsweise Erysiphe graminis; Sphaerotheca-Arten, wie beispielsweise Sphaerotheca fuliginea;

Podosphaera-Arten, wie beispielsweise Podosphaera leucotricha;

Venturia-Arten, wie beispielsweise Venturia inaequalis; Pyrenophora-Arten, wie beispielsweise Pyrenophora teres oder P. graminea (Konidienform: Drechslera, Syn: Helminthosporium);

Cochliobolus-Arten, wie beispielsweise Cochliobolus sativus (Konidienform: Drechslera, Syn: Helminthosporium);

Uromyces-Arten, wie beispielsweise Uromyces appendiculatus:

Puccinia-Arten, wie beispielsweise Puccinia recondita;

Tilletia-Arten, wie beispielsweise Tilletia caries;

Ustilago-Arten, wie beispielsweise Ustilago nuda oder Ustilago avenae;

Pellicularia-Arten, wie beispielsweise Pellicularia sasakii;

Pyriicularia-Arten, wie beispielsweise Pyricularia oryzae;

Fusarium-Arten, wie beispielsweise Fusarium culmorum;

Botrytis-Arten, wie beispielsweise Botrytis cinerea;

Septoria-Arten, wie beispielsweise Septoria nodorum;

Leptosphaeria-Arten, wie beispielsweise Leptosphaeria nodorum;

Cercospora-Arten, wie beispielsweise Cercospora canescens;

Alternaria-Arten, wie beispielsweise Alternaria brassicae;

Pseudocercosporella-Arten, wie beispielsweise Pseudocercosporella herpotrichoides;

Sclerotinia-Arten, wie z.B. Sclerotinia sclerotiorum.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut, und des Bodens.

Dabei lassen sich die erfindungsgemäßen Wirkstoffe mit besonders gutem Erfolg zur Bekämpfung von Getreidekrankheiten, wie beispielsweise gegen den Erreger der Stengelgrundfäule des Weizens (Fusarium culmorum), zur Bekämpfung von Reiskrankheiten, wie beispielsweise gegen den Erreger der Reisfleckenkrankheit (Pyricularia oryzae) oder zur Bekämpfung von Krankheiten im Obst-und Gemüsebau, wie beispielsweise gegen den Erreger der Graufäule (Botrytis cinerea) einsetzen. Hervorzuheben ist dabei besonders, daß die erfindungsgemäßen Wirkstoffe neben einer guten protektiven Wirksamkeit auch systemische Eigenschaften besitzen und sich daher auch als Saatgutbeizmittel eignen. Hervorzuheben ist weiterhin eine gute und breite in vitro-Wirksamkeit der Verbindungen und eine gute Pflanzenverträglichkeit.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Al kylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon,

Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder -schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Grandulate angewendet werden. Die Anwendung erfolgt in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

## Herstellungsbeispiele

Beispiel 1:

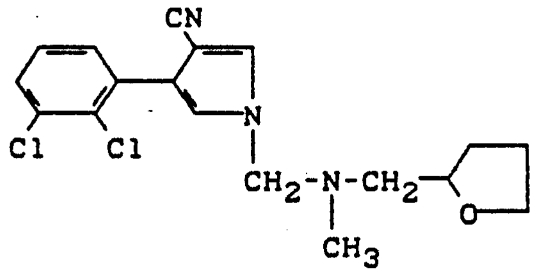

(Verfahren a)

Zu 4,0 g (0,017 Mol) 4-Cyano-3-(2,3-dichlorphenyl)-pyrrol (vgl. EP 133 247) in 14 ml Ethanol gibt man 2 ml (0,027 Mol) 37-prozentige wässrige Formaldehydlösung und 0,16 ml Essigsäure. Danach gibt man tropfenweise unter Rühren 3,0 g (0,027 Mol) N-Methyltetrahydrofurfurylamin (vgl. Ital. Pat. 510, 118 vom 20.1.1955 bzw. CA 52 : 17 287 i) zu und rührt nach beendeter Zugabe 20 Stunden bei Raumtemperatur. Zur Aufarbeitung gibt man 100 ml Ethylacetat zu, wäscht dreimal mit Wasser, trocknet über Natriumsulfat, entfernt das Lösungsmittel im Vakuum und kristallisiert den Rückstand aus Ether/Cyclohexan.

Man erhält 4,0 g (66 % der Theorie) an 4-Cyano-3-(2,3-dichlorphenyl)-1-(N-methyl-N-furfuryl-aminomethyl)-pyrrol vom Schmelzpunkt 79 °C - 80 °C.

In entsprechender Weise und gemäß den allgemeinen Angaben zur Herstellung erhält man die folgenden 1-Amino-methyl-3-aryl-4-cyano-pyrrole der allgemeinen Formel (I):

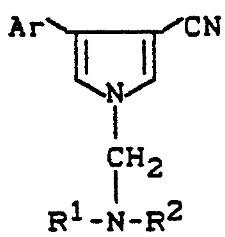

(I)

| Bsp. Nr. | Ar | $R^1$ | $R^2$ | physikalische Eigenschaften |
|---|---|---|---|---|
| 2 | 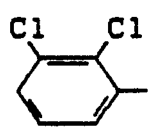 | $CH_3$ | 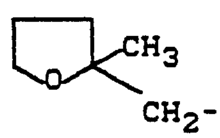 | $^1H$-NMR*): 4,75-5,0 |
| 3 | 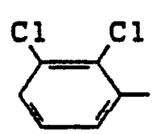 | n-$C_3H_7$ | 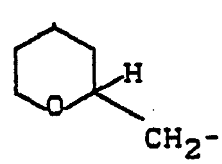 | Fp. 67-68° C |
| 4 | 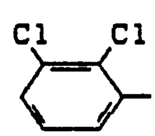 | $NC-CH_2-CH_2-$ | $(C_2H_5)_2N-(CH_2)_2-$ | $^1H$-NMR*):4,9 |

| Bsp. Nr. | Ar | $R^1$ | $R^2$ | physikalische Eigenschaften |
|---|---|---|---|---|
| 5 | 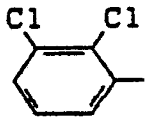 | $CH_3$ | 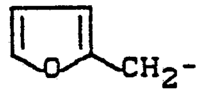 | Fp. 60-61° C |
| 6 | 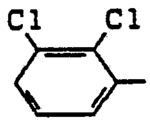 | $n-C_3H_7$ | 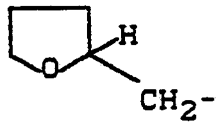 | $^1H$-NMR*): 4,8-5,1 |
| 7 | 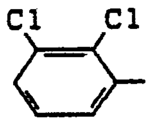 | $NC-CH_2-CH_2-$ | 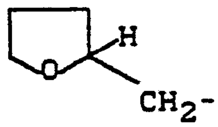 | $^1H$-NMR*): 4.85-5,15 |
| 8 | 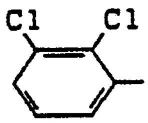 | $NC-CH_2-CH_2-$ | 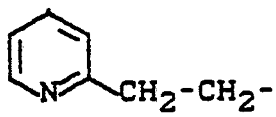 | $^1H$-NMR*): 4,8 |
| 9 | 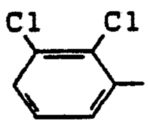 | 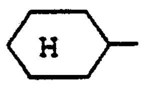 | 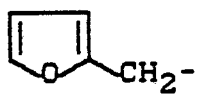 | Fp. 70-71° C |
| 10 | 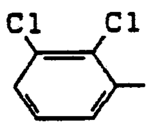 | $CH_3$ | 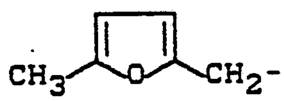 | Fp. 68-70° C |
| 11 | 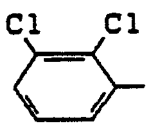 | $NC-CH_2-CH_2-$ | 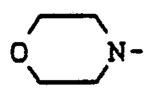 $-(CH_2)_2-$ | Fp.79-80° C |
| 12 | 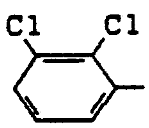 | $C_2H_5$ | 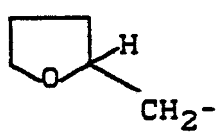 | $^1H$-NMR*): 4,8-5,1 |

| Bsp. Nr. | Ar | $R^1$ | $R^2$ | physikalische Eigenschaften |
|---|---|---|---|---|
| 13 | 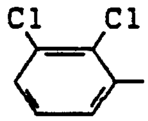 | $s-C_4H_9$ | 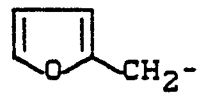 | Fp. 40-43° C |
| 14 | 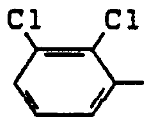 | $CH_3$ | 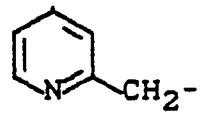 | Fp. 65-66° C |
| 15 | 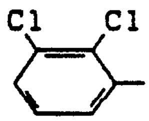 | $i-C_4H_9$ | 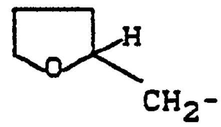 | Fp 64-65° C |
| 16 | 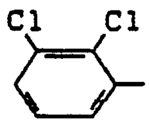 | $NC-CH_2-CH_2-$ | 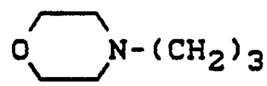 | Fp.62-63° C |
| 17 | 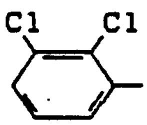 | $i-C_3H_7$ | 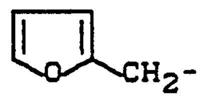 | $^{1}H$-NMR*): 4,65 |
| 18 | 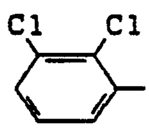 | $CH_3$ | 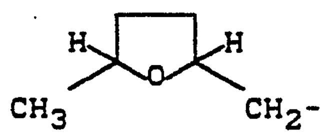 | $^{1}H$-NMR*): 4,8-5,0 |
| 19 | 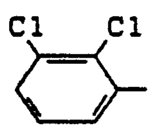 | 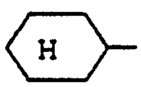 | 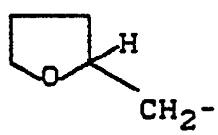 | $^{1}H$-NMR*): 4,8-5,0 |
| 20 | 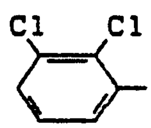 | $NC-CH_2-CH_2-$ | $(CH_3)_2N-(CH_2)_3-$ | $^{1}H$-NMR*): 4,8 |

| Bsp. Nr. | Ar | $R^1$ | $R^2$ | physikalische Eigenschaften |
|---|---|---|---|---|
| 21 | 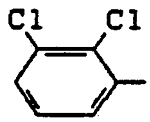 | $NC-CH_2-CH_2-$ | $(C_2H_5)_2N-(CH_2)_3-$ | $^1H$-NMR*): 4,85 |
| 22 | 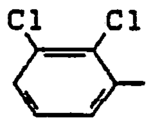 | $NC-CH_2-CH_2-$ | $CH_3$<br>$(C_2H_5)_2N-(CH_2)_3-CH-$ | $^1H$-NMR*): 4,7-4,9 |
| 23 | 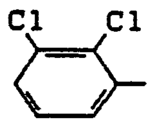 | $NC-CH_2-CH_2-$ | 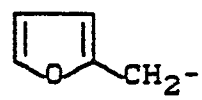 | $^1H$-NMR*): 4,8 |
| 24 | 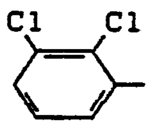 | 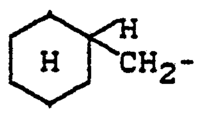 | 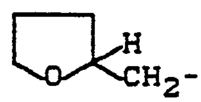 | Fp. 84-85° C |
| 25 | 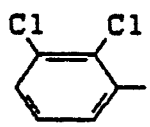 | $NC-CH_2-CH_2-$ | $(n-C_4H_9)_2N-(CH_2)_3-$ | $^1H$-NMR*): 4,8 |
| 26 | 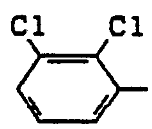 | $NC-CH_2-CH_2-$ | 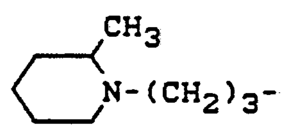 | Fp.57-59° C |
| 27 | 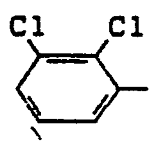 | $CH_3$ | 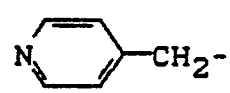 | $^1H$-NMR*): 4,8 |
| 28 | 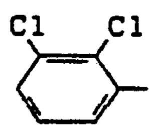 | $CH_3$ | 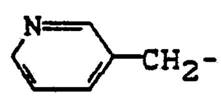 | $^1H$-NMR*): 4,8 |

| Bsp. Nr. | Ar | $R^1$ | $R^2$ | physikalische Eigenschaften |
|---|---|---|---|---|
| 29 | 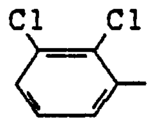 | $C_2H_5$ | 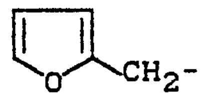 | $^1$H-NMR*): 4,75 |
| 30 | 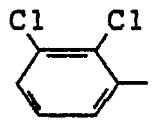 | $C_3H_7$-n | 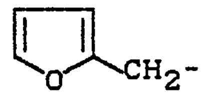 | $^1$H-NMR*): 4,70 |
| 31 | 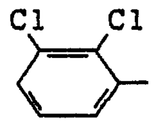 | NC-$CH_2CH_2$- | 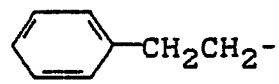 | Fp. 69-70° C |
| 32 | 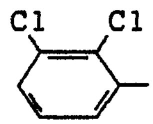 | NC-$CH_2CH_2$- | 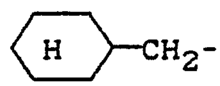 | $^1$H-NMR$^{+)}$: 4,80 |
| 33 | 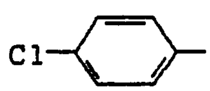 | $CH_3$ | 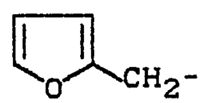 | $^1$H-NMR*): 4,70 |
| 34 | 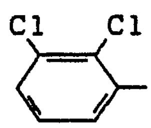 | $C_3H_7$-n | 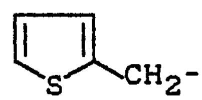 | Fp. 86-87° C |
| 35 | 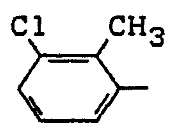 | $CH_3$ | 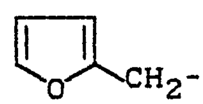 | $^1$H-NMR*): 4,70 |
| 36 | 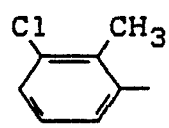 | 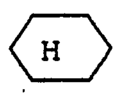 | 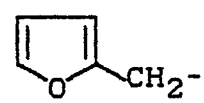 | $^1$H-NMR*): 4,80 |

*) Die $^1$H-NMR-Spektren wurden in $CDCl_3$ mit Tetramethylsilan (TMS) als innerem Standard aufgenommen. Angegeben ist die chemische Verschiebung der

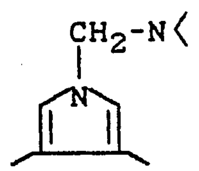 - Gruppe

als δ-wert im ppm.

Anwendungsbeispiele

In den folgenden Anwendungsbeispielen wurden die nachstehend aufgeführten Verbindungen als Vergleichsubstanzen eingesetzt:

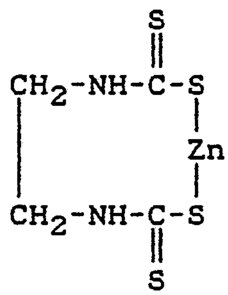

(A)

Zink-ethylen-1,2-bis-(dithiocarbamat);

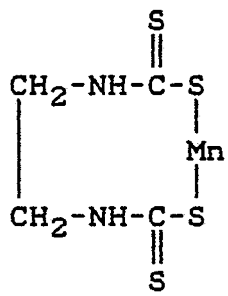

(B)

Mangan-ethylen-1,2-bis-(dithiocarbamat);

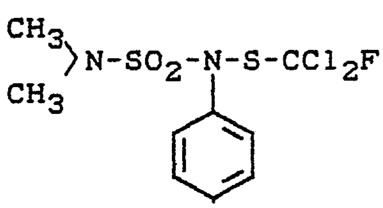

(C)

N,N-Dimethyl-N'-(fluordichlormethylsulfenyl)-sulfamid und

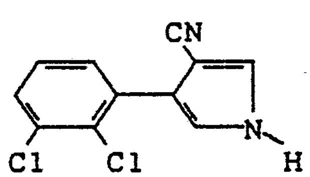

(D)

4-Cyano-3-(2,3-dichlorphenyl)-pyrrol

(bekannt aus EP 174 910).

Beispiel A

Fusarium culmorum-Test (Weizen) / Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Den Weizen sät man mit 2 x 100 Korn 1 cm tief in eine Standarderde und kultiviert ihn im Gewächshaus bei einer Temperatur von ca. 18°C in Saatkästen, die täglich 15 Stunden dem Licht ausgesetzt werden.

Ca. 3 Wochen nach der Aussaat erfolgt die Auswertung der Pflanzen auf Symptome.

Eine deutliche Überlegenheit in der Wirksamkeit gegenüber dem Stand der Technik zeigen bei diesem Test z.B. die Verbindungen gemäß den Herstellungsbeispielen: 2 und 6.

Beispiel B

Botrytis-Test (Bohne)/protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20 °C aufgestellt. 3 Tage nach der Inokulation wird die Größe der Befallsflecken auf den Blättern ausgewertet.

Eine deutliche Überlegenheit in Wirksamkeit gegenüber dem Stand der Technik zeigen in diesem Test z.B. die Verbindungen gemäß den Herstellungsbeispielen: 1, 2, 3, 5, 7 und 9.

Beispiel C

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeispielen: 1, 4, 7, 8 und 9.

Beispiel D

Pflanzenverträglichkeitstest

Testpflanze: Reben
Versuchsdauer: 6 Tage
Lösungsmittel: 4,7 Gewichtsteiele Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Mit dieser Wirkstoffzubereitung bespritzt man junge Pflanzen bis zur Tropfnässe und stellt sie in einem Gewächshaus bei ca. 20 °C auf.

Die Pflanzen werden auf Schäden wie Wuchsbeeinträchtigungen, Verfärbungen und Nekrosen ausgewertet. Nach den angegebenen Zeiten wird der Schädigungsgrad per Pflanzen bestimmt.

Eine deutliche Überlegenheit in der Pflanzenverträglichkeit gegenüber dem Stand der Technik zeigen bei diesem Test z.B. die Verbindungen gemäß den Herstellungsbeispielen: 5 und 9.

## Ansprüche

1. 1-Aminomethyl-3-aryl-4-cyano-pyrrole der allgemeinen Formel (I),

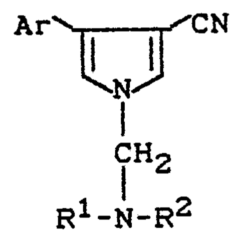

(I)

in welcher

Ar für gegebenenfalls substituiertes Phenyl steht,

$R^1$ für gegebenenfalls substituiertes Alkyl, für Alkenyl, Alkinyl oder Cycloalkyl oder für jeweils gegebenenfalls substituiertes Aralkyl oder Aryl steht und

$R^2$ für jeweils gegebenenfalls substituiertes Heterocyclylalkyl, Heterocyclylalkenyl, Heterocyclylalkinyl oder Heterocyclyl, für Dialkylaminoalkyl oder für jeweils gegebenenfalls substituiertes Cycloalkylalkyl oder Phenethyl steht.

2. 1-Aminomethyl-3-aryl-4-cyano-pyrrole gemäß Anspruch 1, wobei in der Formel (I)

Ar für gegebenenfalls einfach bis mehrfach, gleich oder verschieden substituiertes Phenyl steht, wobei als Substituenten infrage kommen: Halogen, jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^1$ für gegebenenfalls substituiertes, geradkettiges oder verzweigtes Alkyl mit 1 bis 6 Kohlenstoffatomen steht, wobei als Substituenten infrage kommen: Cyano, Cycloalkyl mit 3 bis 7 Kohlenstoffatomen, je geradkettiges oder verzweigtes Alkoxy, Alkylcarbonyl, Alkoxycarbonyl, Alkylaminocarbonyl, Dialkylaminocarbonyl, Alkylsulfinyl, Alkylsulfonyl oder Dialkylamino mit jeweils 1 bis 6 Kohlenstoffatomen in den einzelnen Alkylteilen; außerdem für jeweils geradkettiges oder verzweigtes Alkenyl oder Alkinyl mit jeweils 3 bis 6 Kohlenstoffatomen, für Cycloalkyl mit 3 bis 7 Kohlenstoffatomen oder für jeweils gegebenenfalls einfach bis mehrfach, gleich oder verschieden im Phenylteil substituiertes Phenyl oder Benzyl steht, wobei als Substituenten jeweils infrage kommen:

Halogen, Cyano, jeweils geradkettiges oder verzweigtes Alkyl, Alkoxy, Halogenalkyl, Halogenalkoxy, Alkylcarbonyl oder Alkoxycarbonyl mit jeweils 1 bis 6 Kohlenstoffatomen in den einzelnen Alkylteilen und im Fall des Halogenalkyl-oder Halogenalkoxy-Restes mit jeweils 1 bis 9 gleichen oder verschiedenen Halogenatomen und

$R^2$ für jeweils geradkettiges oder verzweigtes Heterocyclylalkyl mit 1 bis 6 Kohlenstoffatomen in Alkylteil, Heterocyclylalkenyl mit 3 bis 6 Kohlenstoffatomen im Alkenylteil, Heterocyclylalkinyl mit 3 bis 6 Kohlenstoffatomen im Alkinylteil oder Heterocyclyl steht, wobei Heterocyclyl jeweils für einen 5-bis 7-gliedrigen, gegebenenfalls einfach bis mehrfach, gleich oder verschieden substituierten, ungesättigen oder gesättigten Heterocyclus mit 1 bis 3 Heteroatomen steht und wobei als Subsktituenten jeweils infrage kommen: Halogen, Cyano, jeweils geradkettiges oder verzweigtes Alkyl, Alkoxy, Halogenalkyl, Halogenalkoxy, Alkylcarbonyl oder Alkoxycarbonyl mit jeweils 1 bis 6 Kohlenstoffatomen in den einzelnen Alkylteilen und im Fall des Halogenalkyl-oder Halogenalkoxy-Restes mit jeweils 1 bis 9 gleichen oder verschiedenen Halogenatomen;

außerdem für geradkettiges oder verzweigtes Dialkylaminoalkyl mit jeweils 1 bis 6 Kohlenstoffatomen in den

einzelnen Alkylteilen steht, ferner für im Cycloalkylteil gegebenenfalls einfach bis mehrfach, gleich oder verschieden durch Halogen und geradkettiges oder verzweigtes Alkyl mit 1 bis 4 Kohlenstoffatomen substituiertes Cycloalkylalkyl mit 1 bis 6 Kohlenstoffatomen im geradkettigen oder verzweigten Alkylteil und 3 bis 7 Kohlenstoffatomen im Cycloalkylteil oder für im Phenyteil gegebenenfalls einfach bis mehrfach, gleich oder verschieden durch Cyano, Halogen und jeweils geradkettiges oder verzweigtes Alkyl oder Alkoxy mit jeweils 1 bis 4 Kohlenstofflatomen substituiertes Phenylethyl steht.

3. 1-Aminomethyl-3-aryl-4-cyano-pyrrole gemäß Anspruch 1, wobei in der Formel (I)
Ar für gegebenenfalls ein-bis dreifach, gleich oder verschieden substituiertes Phenyl steht, wobei als Substituenten infrage kommen: Fluor, Chlor, Brom, Methyl, Ethyl, n-oder i-Propyl, n-, i-, s-oder t-Butyl, Methoxy, Ethoxy, Methylthio, Trifluormethyl, Trifluormethoxy oder Trifluormethylthio,
$R^1$ für jeweils gegebenenfalls substituiertes Methyl, Ethyl oder n-oder i-Propyl sowie n-, i-oder s-Butyl steht, wobei als Substituenten jeweils infrage kommen: Cyano, Methoxy, Ethoxy, Methylcarbonyl, Ethylcarbonyl, Methoxycarbonyl, Ethoxycarbonyl, Methylaminocarbonyl, Dimethylaminocarbonyl, Ethylaminocarbonyl, Diethylaminocarbonyl, Methylsulfinyl, Methylsulfonyl, Dimethylamino, Diethylamino oder Dipropylamino, Dibutylamino oder Cyclohexyl; außerdem für Allyl, n-oder i-Butenyl, Propargyl, n-oder i-Butinyl, für Cyclopentyl, Cyclohexyl, Cyclopropyl oder für jeweils gegebenenfalls ein-bis dreifach, gleich oder verschieden im Phenylteil substituiertes Benzyl oder Phenyl steht, wobei als Substituenten infrage kommen: Fluor, Chlor, Brom, Cyano, Methyl, Ethyl, n-oder i-Propyl, n-, i-oder s-Butyl, Methoxy, Ethoxy, Trifluormethyl, Trifluormethoxy, Acetyl, Methoxyclarbonyl oder Ethoxycarbonyl und
$R^2$ für jeweils gegebenenfalls im Heterocyclylteil ein-oder zweifach, gelcih oder verschieden substituiertes Heterocyclylmethyl, Heterocyclylethyl oder Heterocyclylpropyl steht, wobei als Heterocyclylrest jeweils einer der folgenden Reste infrage kommt

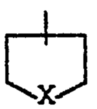 ; 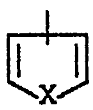 ; 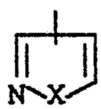 ; 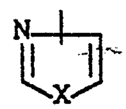 ; 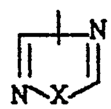 ; 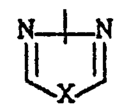

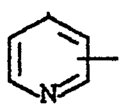 ; 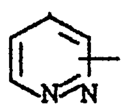 ; 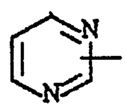 ; 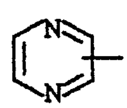 ; 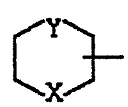

und 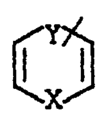 ,

wobei X jeweils für Sauerstoff, Schwefel oder eine NH-Gruppe steht und Y jeweils für Sauerstoff, Schwefel, eine NH-Gruppe oder eine $CH_2$-Gruppe steht und wobei als Substituenten jeweils infrage kommen: Fluor, Chlor, Brom, Cyano, Methyl, Ethyl, n-oder i-Propyl, n-, i-, s-oder t-Butyl, Methoxy, Ethoxy, Trifluormethyl, Trifluormethoxy, Acetyl, Methoxycarbonyl oder Ethoxycarbonyl; außerdem für Dimethylaminoethyl, Diethylaminoethyl, Dimethylaminopropyl, Diethylaminopropyl, Dipropylaminopropyl, Dibutylaminopropyl, Dibutylaminobutyl, Dibutylaminoethyl, Dipropylaminoethyl, Dimethylaminobutyl, Diethylaminobutyl, Dimethylaminopentyl, Diethylaminopentyl, für jeweils gegebenenfalls ein-bis dreifach, gleich oder verschieden durch Methyl, Ethyl und Isopropyl substituiertes Cyclohexylmethyl oder Cyclohexylethyl oder für gegebenenfalls ein-bis dreifach, gleich oder verschieden durch Fluor, Chlor, Brom, Cyano, Methyl, Ethyl, n-oder i-Propyl, n-, i-, s-oder t-Butyl, Methoxy, Ethoxy, n-oder i-Propoxy und n-, i-, s-oder t-Butoxy substituiertes Phenethyl steht.

4. 1-Aminomethyl-3-aryl-4-cyano-pyrrole gemäß Anspruch 1, wobei in der Formel (I)
Ar für zweifach durch Chlor substituiertes Phenyl steht,
$R^1$ für Methyl, Ethyl, n-oder i-Propyl, n-, i-oder s-Butyl, Allyl, Propargyl, für Cyclohexyl, Cyclopropyl, Cyclopentyl, Cyclohexylmethyl, Phenyl, Benzyl oder für Cyanethyl steht und
$R^2$ für jeweils gegebenenfalls ein-bis dreifach, gleich oder verschieden durch Chlor und Methyl substituiertes Heterocyclylmethyl, Heterocyclylethyl oder Heterocyclylpropyl steht, wobei als Heterocyclylreste infrage kommen:

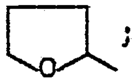 ; 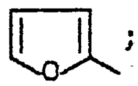 ; 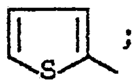 ; 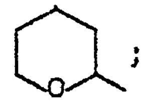 ; 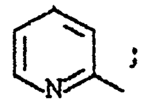 ;

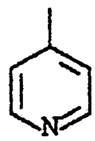 oder 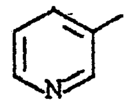 ;

oder für jeweils gegebenenfalls ein-bis dreifach, gleich oder verschieden durch Methyl und Ethyl substituiertes 2-Heterocycylethyl, 2-Heterocyclylpropyl oder 3-Heterocyclylpropyl steht, wobei als Heterocyclylreste infrage kommen:

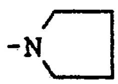 ; 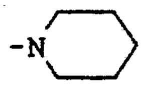 ; 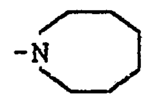 oder 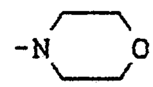 ;

außerdem für Diethylaminoethyl, Dimethylaminopropyl, Diethylaminopropyl, Dibutylaminopropyl oder Diethylaminopentyl, für Cyclohexylmethyl oder für Phenethyl steht.

5. 1-Aminomethyl-3-aryl-4-cyano-pyrrole gemäß Anspruch 1, wobei in der Formel (I)
Ar für 2,3-Dichlorphenyl steht,
$R^1$ für Cyanethyl steht und
$R^2$ für jeweils gegebenenfalls ein-bis dreifach, gleich oder verschieden durch Chlor und Methyl substituiertes Heterocyclylmethyl, Heterocyclylethyl oder Heterocyclylpropyl steht, wobei als Heterocyclylreste infrage kommen:

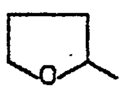 ; 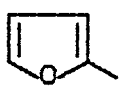 ; 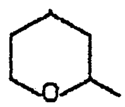 ; 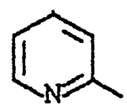 ; 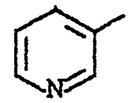

oder 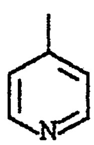 ;

oder für jeweils gegebenenfalls ein-bis dreifach, gleich oder verschieden durch Methyl und Ethyl substituiertes 2-Heterocyclylethyl, 2-Heterocyclylpropyl oder 3-Heterocyclylpropyl steht, wobei als Heterocyclylreste infrage kommen:

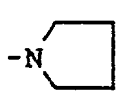 ; 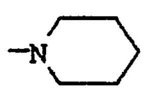 ; 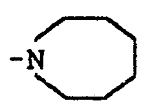 oder 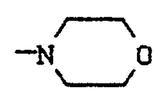 ;

außerdem für Diethylaminoethyl, Dimethylaminopropyl, Diethylaminopropyl, Dibutylaminopropyl oder Diethylaminopentyl, für Cyclcohexylmethyl oder Phenethyl steht.

6. 1-Aminomethyl-3-aryl-4-cyano-pyrrole gemäß Anspruch 1, wobei in der Formel (I)
Ar für 2,3-Dichlorphenyl steht,
$R^1$ für Methyl, Ethyl, n-oder i-Propyl, n-, i-oder s-Butyl, Allyl, Propargyl, für Cyclopropyl, Cyclopentyl, Cyclohexyl, Cyclohexylmethyl, Phenyl, Benzyl oder Cyanoethyl steht und
$R^2$ für gegebenenfalls ein-bis dreifach, gleich oder verschieden durch Chlor und Methyl substituiertes Heterocyclylmethyl steht, wobei als Heterocyclylreste infrage kommen:

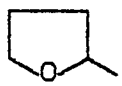 ; 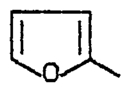 ; 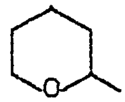 ; 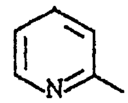 ; 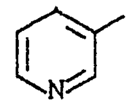

oder 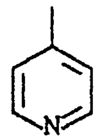 .

7. Verfahren zur Herstellung von 1-Aminomethyl-3-aryl-4-cyano-pyrrolen der allgemeinen Formel (I)

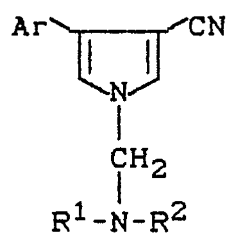

(I)

in welcher
Ar für gegebenenfalls substituiertes Phenyl steht,
$R^1$ für gegebenenfalls substituiertes Alkyl, für Alkenyl, Alkinyl oder Cycloalkyl oder für jeweils gegebenenfalls substituiertes Aralkyl oder Aryl steht und
$R^2$ für jeweils gegebenenfalls substituiertes Heterocyclylalkyl, Heterocyclylalkenyl, Heterocyclylalkinyl oder Heterocyclyl, für Dialkylaminoalkyl oder für jeweils gegebenenfalls substituiertes Cycloalkylalkyl oder Phenethyl steht,
dadurch gekennzeichnet, daß man
(a) 3-Aryl-4-cyano-pyrrole der Formel (II),

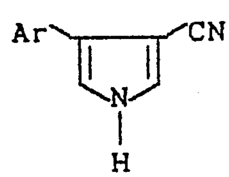

(II)

in welcher
Ar die oben angegebene Bedeutung hat,
mit Formaldehyd und Aminen der Formel (III),

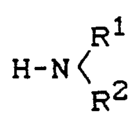

(III)

in welcher
$R^1$ und $R^2$ die oben angegebene Bedeutung haben,
gegebenenfalls in Gegenwart eines Verdünnungsmittels und gegebenenfalls in Gegenwart eines Reaktionshilfsmittels umsetzt, oder
(b) 3-Aryl-4-cyano-pyrrole der Formel (IV),

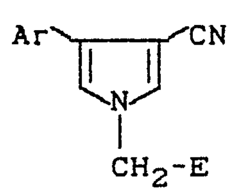

(IV)

in welcher
Ar die oben angegebene Bedeutung hat und
E für eine elektronenanziehende Abgangsgruppe steht,
mit Aminen der Formel (III),

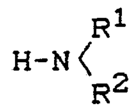 (III)

in welcher
$R^1$ und $R^2$ die oben angegebene Bedeutung haben,
gegebenenfalls in Gegenwart eines Verdünnungsmittels und gegebenenfalls in Gegenwart eines Säurebindemittels umsetzt.

8. Schädlingsbekämpfungsmittel, gekennzeichnet durch einen Gehalt an mindestens einem 1-Aminomethyl-3-aryl-4-cyano-pyrrol der Formel (I) nach den Ansprüchen 1 und 7.

9. Verwendung von 1-Aminomethyl-3-aryl-4-cyano-pyrrolen der Formel (I) nach den Ansprüchen 1 und 7 zur Bekämpfung von Schädlingen.

10. Verfahren zur Bekämpfung von Schädlingen, dadurch gekennzeichnet, daß man 1-Aminomethyl-3-aryl-4-cyano-pyrrole der Formel (I) nach den Ansprüchen 1 und 7 auf Schädlinge und/oder ihren Lebensraum einwirken läßt.

11. Verfahren zur Herstellung von Schädlingsbekämpfungsmitteln, dadurch gekennzeichnet, daß man 1-Aminomethyl-3-aryl-4-cyano-pyrrole der Formel (I) nach den Ansprüchen 1 und 7 mit Streckmitteln und/oder oberflächenaktiven Mitteln vermischt.